(19)

Europäisches
Patentamt

European
Patent Office

Office européen
des brevets

(11) **EP 4 257 980 A1**

(12) **EUROPEAN PATENT APPLICATION**

(43) Date of publication:
**11.10.2023 Bulletin 2023/41**

(21) Application number: **22166440.2**

(22) Date of filing: **04.04.2022**

(51) International Patent Classification (IPC):
**G01N 33/543** (2006.01)

(52) Cooperative Patent Classification (CPC):
**G01N 33/54333**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sensirion AG**
**8712 Stäfa (CH)**

(72) Inventors:
- **Streiff, Matthias**
  **Zürich (CH)**
- **Huber, Deborah**
  **Zürich (CH)**
- **Bürgi, Lukas**
  **Zürich (CH)**
- **Keller, Dino**
  **Männedorf (CH)**

(54) **MAGNETIC IMMUNOASSAY DEVICE**

(57) The present invention relates to a method and a device for determining a bond strength of functionalized magnetic labels (1) to a functionalized detection surface (2) in an immunoassay. The labels (1) are transported to the surface (2) for an incubation step. Subsequently, the labels (1) are manipulated by a magnetic force field and initial positions and dissociation times from the surface (2) of the labels (1) are recorded. These dissociation times are then compared to pre-computed time-spans per position, the time-spans chosen such that the probability of dissociation of a label (1) not specifically bound at a position before said time span has elapsed is greater than a predetermined threshold. If the dissociation time is greater than said time span, the label (1) is designated as specifically bound to the surface (2).

Figure 1

**EP 4 257 980 A1**

## Description

## Technical Field

The present invention relates to a magnetic immunoassay device and corresponding method for determining a type of binding of functionalized beads to a functionali zed detection surface using a force field.

## Background

Magnetic immunoassays are known. A receptacle with a fluid sample comprising a target analyte is provided with labels that comprise magnetic beads. The magnetic labels are functionalized on their surface with affinity bodies that are chosen on account of specifically binding to the target analyte. In a first incubation step, specific binding between the affinity bodies and the target analyte is facilitated to yield analyte-label-complexes.

In a transportation step, the analyte-label-complexes, and free labels if there remain such, are transported to a read-out location, which is preferably in the receptacle and that may comprise a detection surface delimiting the interior of the receptacle at a bottom part of the receptacle in relation to a direction of gravity. The detection surface is itself functionalized with affinity bodies for specific binding to the target analyte. The affinity bodies on the detection surface may be the same as the ones on the magnetic labels or different ones, depending on which epitope of the target analyte the respective affinity body preferentially binds to.

In a second incubation step, the analyte-label-complexes may bind in sandwich format to the detection surface in the format: detection surface-affinity body-target analyte-affinity body-magnetic label.

In a washing step, a magnetic, electrical, fluidic or any other suitable manipulating force is applied to the magnetic labels to distinguish between specifically bound and non-specifically bound magnetic labels on the detection surface. Here, specifically bound may be understood as a relatively strong lock-and-key bond between affinity body on the detection surface and magnetic label bound thereto via target analyte and corresponding functionalization, whereas non-specifically bound may be taken to mean free magnetic labels, that is, magnetic labels that are unattached to any target analyte molecules, adhering to the detection surface via relatively weak interaction forces. The manipulating force selectively removes the non-specifically bound magnetic labels because a specifically bound magnetic label will need a higher threshold force to detach from the surface than a non-specifically bound one, because the bond strength of a specifically bound magnetic label is relatively higher. Finally, the number or proportion of magnetic labels specifically bound to the detection surface is counted, and the count is compared to a prior calibration measurement to determine the target analyte concentration.

Above, the sandwich-capture format has been described. Other assay formats exist such as competitive assays where unlabeled analyte competes with labeled analyte for the affinity bodies, for example. Such assay formats may share with the above-described sandwich-capture format one or more of the steps of incubation-transport-incubation-washing-readout.

Above, the different strengths of specific and non-specific bonds that may facilitate distinguishing between them by applying a manipulating force have been described. However, both bonds are of non-covalent type, such as electrostatic interactions, van-der-Waals forces, hydrogen bonds, and may differ only quantitatively, each bond having a characteristic dissociation rate depending on the manipulating force. In particular, the dissociation rate of any bond is non-zero even for vanishing manipulating force. The dissociation rate at zero manipulating force may be referred to as thermal dissociation rate.

US6180418B1 discloses a sensor for a selected target species comprising a functionalized substrate, magnetically active functionalized beads, an adjustable magnetic field source for exerting a force on the beads, and an imaging system for observing and counting the beads bound to the substrate. When a magnetic field is applied to the substrate the magnetic beads will be pulled away from the substrate unless they are specifically bound, indicating presence of the target species.

WO2007141691A3 discloses a microelectronic sensor device for measuring properties of a target substance in a sample fluid comprising a sensor region where the target substance can be immobilized, a washing unit for moving magnetically interactive washing particles such that a flow of washing particles and/or sample fluid is induced through the sensor region to wash away non-specifically bound target substance. The flow is induced by driving current sequentially through a series of parallel wires in the washing unit thereby generating a time-varying magnetic force field.

WO2012048288A1 discloses a biosensor for detecting and manipulating magnetic particles comprising a light source and an integrated circuit containing optical sensors. Particles on the surface of the integrated circuit cast a shadow that changes the amount of light from the light source that is received by the optical sensors. A washing step is disclosed where non-specifically bound magnetic particles are removed by magnetic separation field generators embedded in the integrated circuit.

In the performance of magnetic immunoassays to determine a target analyte concentration and/or its presence in a fluid sample as described above, it may be a challenge to eradicate false positives, that is, where non-specifically bound labels are erroneously classified as specifically bound ones in relation to a functionalized surface. This may cause a limited accuracy with which the target analyte concentration is determined. It is, therefore, a goal of the present invention to enable determining a target analyte concentration in a fluid sample with improved accuracy by providing a magnetic immunoassay method and an immunoassay device that determine with increased fidelity if a magnetic label is specifically or not specifically bound to a surface.

**Summary of the invention**

According to an embodiment of a first aspect of the present invention, there is provided a method for determining at least a type of binding of functionalized magnetic labels to a functionalized detection surface in a magnetic immunoassay comprising the steps of: providing a sample fluid to a receptacle whose interior is at least partly delimited by the functionalized detection surface, wherein the sample fluid comprises a target analyte and the functionalized magnetic labels, facilitating a first incubation step in which at least a respective proportion of the functionalized magnetic labels and the target analyte form target analyte-magnetic label complexes, or at least part of the target analyte in the sample fluid binds to the functionalized detection surface, transporting at least the magnetic label complexes to the functionalized detection surface, facilitating a second incubation step that is performed for a predetermined incubation time in respect of at least the magnetic label complexes binding to the functionalized detection surface, the method further comprising the steps of: starting a timer determining a time 0 in response to a completion of the predetermined incubation time for at least a magnetic label, manipulating the magnetic label with a predetermined force field for a predetermined manipulation time $t_m$, in response to a commencement of time 0, recording, for the magnetic label, a position $x$ on the functionalized detection surface at time 0, and a time $t_d$ when the magnetic label dissociates from the functionalized detection surface, or recording $t_d = t_m$ if it does not dissociate, computing a time span $t_{el}(x)$ associated to position $x$ for the magnetic label on the functionalized detection surface, and designating the magnetic label as specifically bound to the functionalized detection surface if $t_d > t_{el}(x)$.

In previously-proposed magnetic immunoassays, the determination of the type of binding of functionalized magnetic labels to a functionalized detection surface is performed globally for all magnetic labels by applying a manipulating force and recording if a magnetic label is washed away or not. In an embodiment of the present invention, the determination of the type of binding of functionalized magnetic labels to a functionalized detection surface may be performed for the magnetic labels individually. This has the advantage of improved accuracy of discrimination between specifically bound magnetic labels and non-specifically bound magnetic labels by taking into account the time dependence and position dependence of a manipulating force. Even more advantageously may be taking into account the fact that a magnetic label bound to a surface has a force-dependent, characteristic dissociation rate rather than a fixed threshold force for dissociation.

Preferably, the time span $t_{el}(x)$ is defined such that a probability of dissociation of a magnetic label non-specifically bound at position $x$ before $t_{el}(x)$ has elapsed is greater than a predetermined threshold.

This may provide the advantage of facilitating a type of binding of magnetic labels bound to the functionalized detection surface to be determined with a relatively higher probability.

Desirably, the predetermined force field comprises a magnetic force field.

The magnetic nature of the labels may be taken advantage of by using a correspondent magnetic manipulation force.

Preferably, the predetermined threshold is 63%, preferably 99%.

An increased threshold corresponds to a higher confidence of distinguishing specifically bound from not specifically bound labels relative to the functionalized detection surface.

Preferably, the method further comprises the steps of: determining the predetermined force field $F(x, t)$ acting on a magnetic label at position $x$ at time $t$, determining a dissociation rate $k(F)$ of a magnetic label non-specifically bound to the detection surface as a function of the force field F acting on the magnetic label, wherein the time span $t_{el}(x)$ is a solution to the equation

$$C = \int_0^{t_{el}(x)} k\big(F(x,t)\big)dt ,$$

wherein C is a positive real number.

The advantage of determining the temporal and spatial evolution of the force field as well as determining the force-dependence of the dissociation rate of a magnetic label non-specifically bound to the functionalized detection surface may be to facilitate solution of the above integral equation for $t_{el}(x)$. The specific form of the integral equation fixes the probability, P, of dissociation of a label not specifically bound before $t_{el}$ has elapsed at P = 1 - $e^{-C}$.

Preferably, the constant C satisfies 1 < C < 5.

A value of C=5 implies P=99.3%.

Preferably, the magnetic force field is generated by parallel wires arranged under the functionalized detection surface and configured to be supplied with current.

This may have the advantage of ease of production and small size of the magnetic immunoassay. A silicon, glass, ceramic, or plastic chip or integrated circuit may form at least part of the receptacle such that one surface of the chip is facing the sample fluid. The surface of the chip facing the sample fluid may form the functionalized detection surface. Metal layers of a standard CMOS stack may be used to manufacture parallel wires for carrying current in the chip. Further circuitry on the chip may be configured to supply a tunable current to each wire when supplied with supply voltage, VDD, such that a well-defined but tunable magnetic force field may be generated.

Preferably, the magnetic label at position x is a member of a subset of the magnetic labels, the subset characterized by having a fixed distance $d_0$ to a current carrying wire.

It may be advantageous to restrict the computation of the time span $t_{el}(x)$ to a subset of the magnetic labels that have the same or a very close value of $t_{el}(x)$. This may for instance be the case for a subset of magnetic labels that experience the same or similar manipulating force because the force field has a certain symmetry. For instance, the force field generated by a wire may be cylindrically symmetrical with the symmetry axis along the wire. The intersection of the cylindrically symmetric force field with the plane spanned by the magnetic labels, which is largely parallel to the functionalized detection surface, has a translational symmetry along the wire. A subset of magnetic labels with fixed distance to the current carrying wire may thus be expected to share a common value of $t_{el}(x)$, and may be sufficient to compute only one value of $t_{el}(x)$ for all magnetic labels of the subset, which may speed up the assay.

A corresponding device aspect is also provided, and so according to an embodiment of a second aspect of the present invention, there is provided a magnetic immunoassay device for determining a type of binding of functionalized magnetic labels to a functionalized detection surface, comprising a receptacle whose interior is at least partly delimited by the functionalized detection surface, the receptacle configured to receive a sample fluid comprising a target analyte and the functionalized magnetic labels, means for transporting the magnetic labels to the functionalized detection surface, a timer, a force field source arranged and configured to manipulate magnetic labels bound to the functionalized detection surface, means for determining a position of the magnetic labels bound to the functionalized detection surface, a controller for controlling the timer, the means for determining the position of the magnetic labels, and the force field source, wherein the timer and the controller are configured to perform the steps of method 1.

**Brief description of the drawings**

Reference will now be made to the accompanying drawings in which:

Figure 1 shows a flowchart of performing steps of a process according to an embodiment of the present invention.
Figure 2 schematically shows a magnetic immunoassay device according to an embodiment of the present invention.
Figure 3a shows a magnetostatic simulation of current-carrying wires embedded in the detection surface

Figure 3b illustrates an effect of a manipulating force as applied on a magnetic label in an embodiment of the present invention.

**Description of preferred embodiments**

Within the description, the same reference numerals or signs have been used to denote the same parts or the like.

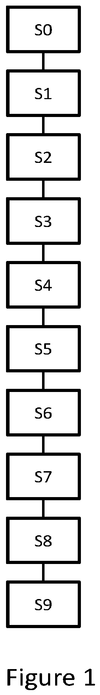

Reference is now made to Figure 1, which shows a flowchart illustrating a method of performing steps of a magnetic immunoassay.

In step S0, a sample fluid comprising the target analyte to be detected / quantified is added to a receptacle together with magnetic labels the surface of which has been functionalized with affinity bodies specific to the target analyte.

In a first incubation step S1, the target analyte binds to the affinity bodies on the magnetic labels to form target-analyte-label-complexes or, in a type of competitive assay, target analyte binds specifically to a functionalized detection surface provided in respect of the receptacle.

In step S2, the magnetic labels are transported to the functionalized detection surface. This may be facilitated by letting the magnetic labels sediment under the force of gravity, for instance, where the functionalized detection surface forms at least a part of a lower boundary of the receptacle with respect to a direction of gravity in an embodiment of the present invention. Active transport of the magnetic labels e.g. using magnetic, electrical, optical, fluidic, or acoustic forces may be preferred if the detection surface during the assay can be oriented in various ways relative to the direction of gravity, or if it is preferred that the sedimentation time be controlled more precisely.

In step S3, the magnetic labels are left on the functionalized detection surface to incubate for a second incubation step. The incubation time may be tailored to the binding kinetics of the affinity bodies on the functionalized detection surface and target analyte in question.

In step S4, a timer is started at time t = 0. The starting time t=0 may be a global starting time, that is, the same for all magnetic labels, or it may be determined individually per magnetic label. The latter may be advantageous if the overall assay duration is desired to be relatively short, and therefore the second incubation time is chosen shorter than would be necessary to reach the equilibrium of the binding kinetics of the affinity bodies on the detection surface and target analyte in question.

In step S5, at time t = 0, positions $x_i$ of the magnetic labels on the detection surface are recorded. Here, the index $i$ enumerates the magnetic labels. This can be done using methods external to the detection surface, e.g. using visible light methods such as photography / videography, infrared illumination from below and detection in case the functionalized detection surface comprises silicon, which is transparent to infrared light, or internal to the detection surface, e.g. Hall effect sensors or magneto-resistance sensors, utilizing the magnetic nature of the labels.

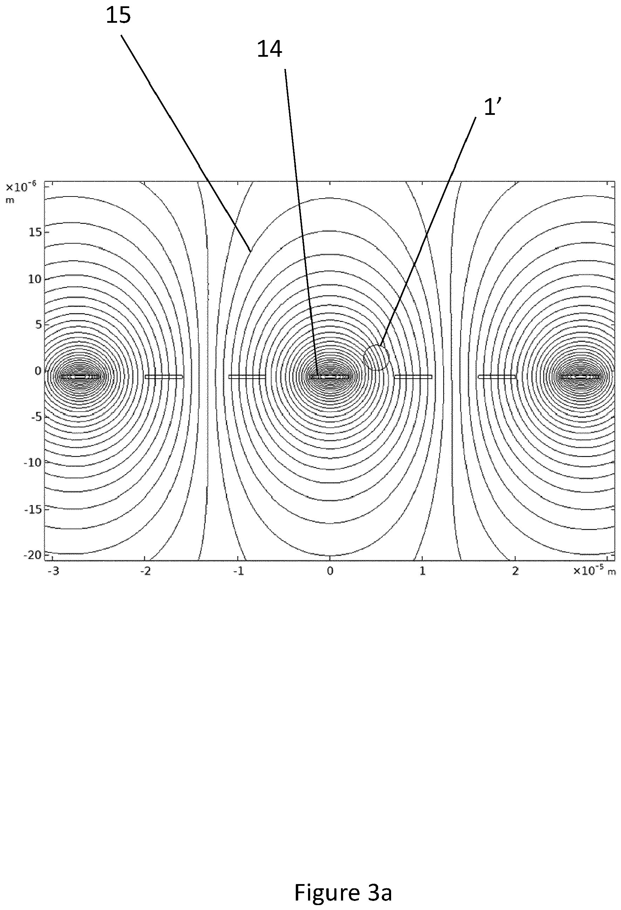

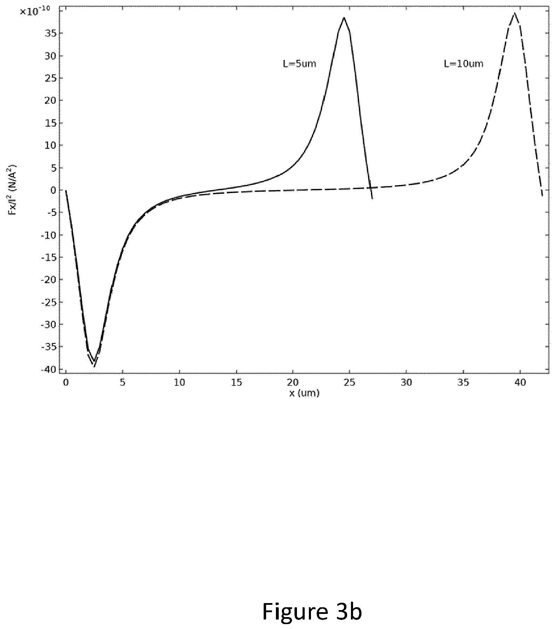

In step S6, starting at time t = 0, the magnetic labels bound to the detection surface are manipulated with a predetermined force field for a predetermined manipulation time. The predetermined force field is chosen such that a manipulating force in the range of 0 and 100pN may be applied. In a preferred embodiment, the manipulating force is magnetic, but other types of forces such as gravitational by inclining the detection surface, electrical, acoustic, or fluidic are also possible. In a most preferred embodiment, the detection surface is a surface of a chip facing the sample fluid, the chip forming at least part of the bottom of the receptacle with regards to the direction of gravity, and there is at least one wire embedded in the chip for carrying an electrical current and thus producing a magnetic field force the strength of which is tunable by the electrical current. By way of example, we show in Figures 3a and 3b how the force field may be predetermined for the case of seven parallel embedded wires of diameter 4 um spaced a distance L = 5 um apart. In Figure 3a, three of the wires 14 are carrying a current of 1mA, in antiparallel configuration. A magnetic label 1 in an embodiment of the present invention is modeled as a paramagnetic metal bead of 2.8 um diameter and a magnetic susceptibility of 0.756. The x-axis is defined to be a direction orthogonal to the wires 14 and in-plane with the wires 14, the z-axis is in a direction normal to the detection surface, the y-axis (not shown) is parallel to the wires 14, and the origin is set at the center of the middle current-carrying wire 14. The vertical position of the bead is fixed at z=0.5 um above the wires' upper surface. Isopotential lines 15 of the z-component of the magnetic vector potential are shown. Figure 3b shows the resulting force in the x-direction in response to the application of the predetermined force field on a bead whose center is located at x-coordinate x, both for an interwire distance of L = 5 um and L = 10 um. The force is proportional to the current squared, so only the force normalized by the current squared is shown. Magnetostatics were simulated with the commercially available software COMSOL™. The highly nonlinear dependence of the resulting force on the distance of the bead 1 from the wires 14. Similarly, the force is dependent on the time if different wires 14 are supplied with current at different times. A prior modeling/measurement of the spatial and temporal evolution of the force field manipulating the labels bound to the detection surface is thus advantageous for increased accuracy in determining the type of binding between the magnetic label 1 relative to the functionalized detection surface.

In step S7, the dissociation times $t_{d,i}$ of the magnetic labels from the detection surface are recorded: The detection surface may be videotaped with a frame rate of 10Hz with a highspeed camera. A magnetic label's movement on the functionalized detection surface is tracked frame by frame by a computer program such as MATLAB®. If a label moves by more than a predetermined amount from its position in the frame at t=0 it is found to have dissociated. If a label does not dissociate from the detection surface during the predetermined manipulation time, this predetermined manipulation time is recorded for the label.

In step S8, a time span $t_{e,i}(x_i)$ is computed for the positions $x_i$ recorded at time $t = 0$, wherein the time span $t_{e,i}(x_i)$ is defined such that the probability of dissociation of a label non-specifically bound at position $x_i$ before $t_{e,i}(x_i)$ has elapsed is greater than a predetermined threshold. Assuming a Poisson distribution, the probability that no dissociation event happens before time $t$ has elapsed is $e^{-kt}$, where $k$ is the dissociation rate of a non-specifically bound label. This dissociation rate, k, and its dependence on a manipulating force is advantageously determined beforehand, e.g. experimentally by running an assay without target analyte and counting the number of dissociation events in a fixed time span and fitting a Poisson distribution with rate constant k for a range of fixed forces.

To simplify the process, it may be advantageous to only compute the time spans $t_{e,i}(x_i)$ for a subset of magnetic labels. For instance, the subset may be magnetic labels that have relatively close time spans $t_{e,i}(x_i)$ because the manipulating force distribution displays some symmetry such as a translational symmetry. In this case, only one time span $t_{e,i}(x)$ needs to be computed, where x is the position of any member of the subset of magnetic labels. An example for a manipulating force with translational symmetry is the one generated by parallel wires 14 carrying current. The translational symmetry axis is along the wires 14.

In step S9, the time span $t_{e,i}(x_i)$ is compared to the recorded dissociation time $t_{d,i}$, and if $t_{d,i} > t_{e,i}(x_i)$ then the magnetic label $i$ is designated as specifically bound, otherwise as non-specifically bound.

A concentration of the target analyte in the fluid sample may be determined by relating the information about the proportion of specifically bound labels vs. non-specifically bound ones to a prior calibration, e.g. by having previously determined this proportion for at least two known concentrations of the target analyte and interpolating appropriately, e.g. linearly.

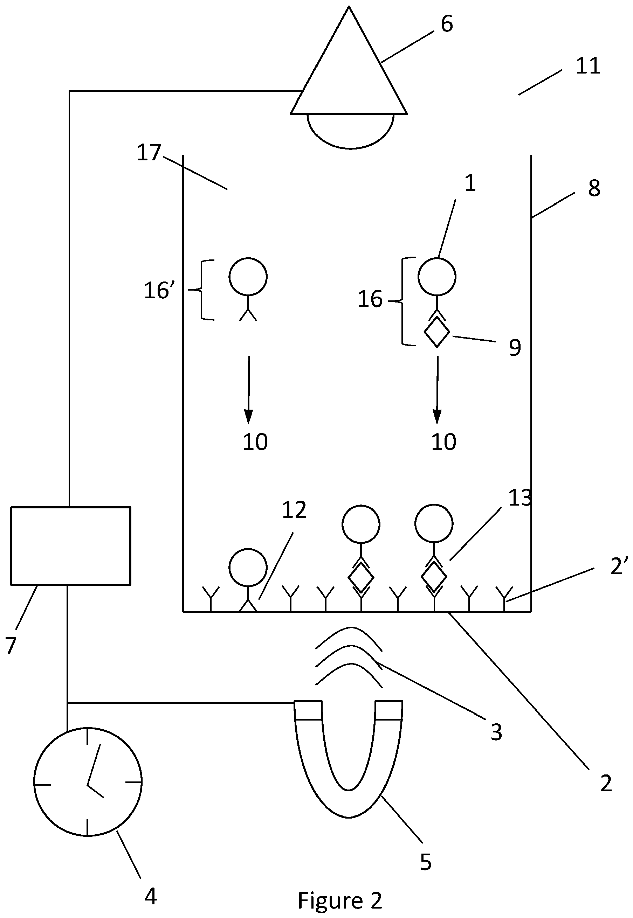

We turn now to Figure 2, which shows a magnetic immunoassay device (11) according to an aspect of the present invention. The device (11) comprises a receptacle (8) comprising fluid with target analyte (9) and functionalized magnetic labels (1), the receptacle being orientable such that gravity-assisted sedimentation (10) of the functionalized magnetic labels (1) to a detection surface (2) constituting the bottom boundary with regard to gravity of the interior of the receptacle is possible. Magnetic labels (1) either bound non-specifically (12) to the detection surface (2) or specifically (13) via lock-and-key interactions between affinity bodies on both surface and label, and the target analyte, may be manipulated with a magnetic force field (3) created by a permanent magnet (5) arranged under the detection surface (2), where the magnet (5) is controlled by controller (7) such as a microcontroller. Also connected to the controller (7) is a timer (4) and a camera (6) for determining the position of a label on the detection surface (2). The controller (7) is configured to execute the steps S2 to S8 described above.

**List of reference numerals**

| 1 | Magnetic Label |
|---|---|
| 1' | Element of a subset of magnetic labels |
| 2 | Detection Surface |
| 2' | Surface functionalization |
| 3 | Force Field |
| 4 | Timer |
| 5 | Force Field Source |
| 6 | Means for determining position of label bound on surface |
| 7 | Controller |
| 8 | Receptacle |
| 9 | Target Analyte |
| 10 | Direction of Gravitation |
| 11 | Immunoassay device |
| 12 | Non-specifically bound label |
| 13 | Specifically bound label |
| 14 | Wire |
| 15 | Isopotential line of z-component of magnetic vector potential |
| 16 | Target analyte-magnetic label complex |
| 16' | free functionalized magnetic label |
| 17 | Sample fluid |

**Claims**

1. A method for determining at least a type of binding of functionalized magnetic labels (1) to a functionalized detection surface (2) in a magnetic immunoassay comprising the steps of:

   providing (S0) a sample fluid (17) to a receptacle (8) whose interior is at least partly delimited by the functionalized detection surface (2), wherein the sample fluid comprises a target analyte (9) and the functionalized magnetic labels (1), facilitating a first incubation step (S1) in which at least a respective proportion of the functionalized magnetic labels (1) and the target analyte (9) form target analyte-magnetic label complexes (16), or at least part of the target analyte (9) in the sample fluid binds to the functionalized detection surface (2), transporting (S2) at least the magnetic label complexes (16) to the functionalized detection surface (2),

   facilitating (S3) a second incubation step that is performed for a predetermined incubation time in respect of at least the magnetic label complexes binding to the functionalized detection surface (2),

   the method further comprising the steps of:

   starting (S4) a timer determining a time 0 in response to a completion of the predetermined incubation time for at least a magnetic label (1), manipulating (S6) the magnetic label (1) with a predetermined force field (3) for a predetermined manipulation time $t_m$, in response to a commencement of time 0, recording (S5, S7), for the magnetic label (1), a position x on the functionalized detection surface (2) at time 0, and a time $t_d$ when the magnetic label (1) dissociates from the functionalized detection surface (2), or recording $t_d = t_m$ if it does not dissociate, computing (S8) a time span $t_{el}(x)$ associated to position x for the magnetic label (1) on the functionalized detection surface (2), and designating (S9) the magnetic label (1) as specifically bound to the functionalized detection surface (2) if $t_d > t_{el}(x)$.

2. The method of claim 1, wherein the time span $t_{el}(x)$ is defined such that a probability of dissociation of a magnetic label (12) non-specifically bound at position x before $t_{el}(x)$ has elapsed is greater than a predetermined threshold.

3. The method of claim 2, wherein the predetermined threshold is 63%, preferably 99%.

4. The method of any preceding claim, wherein the predetermined force field (3) comprises a magnetic force field.

5. The method of claim 4, wherein the magnetic force field (3) is generated by parallel wires (14) arranged under the functionalized detection surface (2) and configured to be supplied with current.

6. The method of claim 5, wherein the magnetic label (1') at position x is a member of a subset of the magnetic labels (1), the subset **characterized by** having a fixed distance $d_0$ to a current carrying wire (14).

7. The method of claim any preceding claim, further comprising the steps of:

   determining the predetermined force field (3) as $F(x,t)$ acting on a magnetic label (1) at position x at time t,
determining a dissociation rate $k(F)$ of a magnetic label (12) non-specifically bound to the functionalized detection surface (2) as a function of the force field F acting on the magnetic label (12),
wherein the time span $t_{el}(x)$ is a solution to the equation

$$C = \int_0^{t_{el}(x)} k\big(F(x,t)\big)dt\,,$$

wherein C is a positive real number.

8. The method of claim 7, wherein the constant C satisfies $1 < C < 5$.

9. A magnetic immunoassay device (11) for determining a type of binding of
functionalized magnetic labels (1) to a functionalized detection surface (2), comprising a receptacle (8) whose interior is at least partly delimited by the functionalized detection surface (2), the receptacle (8) configured to receive a sample fluid (17) comprising a target analyte (9) and the functionalized magnetic labels (1),

> means for transporting the magnetic labels (1) to the functionalized detection surface (2),
> a timer (4),
> a force field source (5) arranged and configured to manipulate magnetic labels (1) bound to the functionalized detection surface (2),
> means (6) for determining a position of the magnetic labels (1) bound to the functionalized detection surface (2),
> a controller (7) for controlling the timer (4), the means for determining the position of the magnetic labels (6), and the force field source (5),
> wherein the timer (4) and the controller (7) are configured to perform the steps of method 1.

```
┌──────────┐
│    S0    │
└──────────┘
      │
┌──────────┐
│    S1    │
└──────────┘
      │
┌──────────┐
│    S2    │
└──────────┘
      │
┌──────────┐
│    S3    │
└──────────┘
      │
┌──────────┐
│    S4    │
└──────────┘
      │
┌──────────┐
│    S5    │
└──────────┘
      │
┌──────────┐
│    S6    │
└──────────┘
      │
┌──────────┐
│    S7    │
└──────────┘
      │
┌──────────┐
│    S8    │
└──────────┘
      │
┌──────────┐
│    S9    │
└──────────┘
```

Figure 1

Figure 2

Figure 3a

Figure 3b

Europäisches
Patentamt

European
Patent Office

Office européen
des brevets

# EUROPEAN SEARCH REPORT

Application Number

EP 22 16 6440

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Danilowicz Claudia ET AL: Analytical chemistry (Washington), 15 May 2005 (2005-05-15), pages 3023-3028, XP055832495, Washington, DC DOI: 10.1021/ac050057+ Retrieved from the Internet: URL:https://pubs.acs.org/doi/pdf/10.1021/ac050057+ [retrieved on 2021-08-17] * page 3026; figure 2 * | 1-9 | INV. G01N33/543 |
| X | JACOB ASHA ET AL: "Quantification of Protein-Ligand Dissociation Kinetics in Heterogeneous Affinity Assays", ANALYTICAL CHEMISTRY, vol. 84, no. 21, 17 October 2012 (2012-10-17), pages 9287-9294, XP055963170, US ISSN: 0003-2700, DOI: 10.1021/ac301894k Retrieved from the Internet: URL:https://pubs.acs.org/doi/pdf/10.1021/ac301894k> * page 9289; figure 2 * | 1-9 | |
| X | JP 2019 020297 A (SYSMEX CORP) 7 February 2019 (2019-02-07) * paragraphs [0006], [0008], [0010], [0011], [0032], [0035], [0037], [0041], [0051], [0077], [0089], [0096] * | 1-9 | TECHNICAL FIELDS SEARCHED (IPC) G01N |
| X | WO 2021/242178 A1 (NAT UNIV SINGAPORE [SG]) 2 December 2021 (2021-12-02) * paragraph [0055] * | 1-9 | |

The present search report has been drawn up for all claims

| Place of search | Date of completion of the search | Examiner |
|---|---|---|
| The Hague | 22 September 2022 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 6440

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2022

| Patent document cited in search report | | Publication date | Patent family member(s) | | Publication date |
|---|---|---|---|---|---|
| JP 2019020297 | A | 07-02-2019 | CN | 109283327 A | 29-01-2019 |
| | | | EP | 3431995 A1 | 23-01-2019 |
| | | | JP | 6949595 B2 | 13-10-2021 |
| | | | JP | 2019020297 A | 07-02-2019 |
| | | | US | 2019025319 A1 | 24-01-2019 |
| WO 2021242178 | A1 | 02-12-2021 | NONE | | |

EPO FORM P0459

For more details about this annex : see Official Journal of the European Patent Office, No. 12/82

**EP 4 257 980 A1**

**REFERENCES CITED IN THE DESCRIPTION**

**Patent documents cited in the description**

- US 6180418 B1
- WO 2007141691 A3
- WO 2012048288 A1